# EUROPEAN PATENT APPLICATION

(11) **EP 4 708 318 A1**
(43) Date of publication of application: **11.03.2026**
(21) Application number: 25199988.4
(22) Date of filing: 03.09.2025
(51) Int. Cl.: G16H 40/20

(54) **SYSTEM AND METHOD FOR IMPROVING PATIENT OUTCOMES WITHIN A SPECIFIC POPULATION**

(30) Priority: 05.09.2024 US 202463690950 P
(71) Applicant: Welch Allyn, Inc., Skaneateles Falls, NY 13153 (US)
(72) Inventor: CORSARO, Theodore F., Skaneateles Falls, 47006 (US)
(74) Representative: Reddie & Grose LLP

(57) **Abstract**

A system and method for improving patient outcomes by dynamically selecting the medical products used for a population to optimize patient outcomes is disclosed.

## Description

### BACKGROUND

The present disclosure is directed to a system and method for improving patient outcomes by dynamically selecting the medical products used for a population to optimize patient outcomes. More specifically, the present disclosure is directed to monitoring the medical profile of a specific population of patients and recursively adjusting the fleet of medical products applied to the population based on the changing medical profile of the population over time.

Care providers in medical settings are regularly required to acquire medical products and to identify particular therapies to provide to patients to manage the patient outcomes at an appropriate cost. Under ongoing scrutiny by payers, including government payers, commercial payers, and consumer payers, care providers are challenged to provide improved patient outcomes at lower costs. This often involves care providers addressing patient needs acquiring and deploying medical equipment and therapeutic devices that provide appropriate care while controlling costs.

Additional pressure is applied as care providers are challenged to undertake risk sharing models with payers, specifically government and commercial payers, such that the reimbursement of care is related to the effectiveness of the care. There is a tension in risk sharing in that care providers are expected to provide a minimum standard of care while also being challenged to increase the effectiveness of the care to prevent recurring patient care, such as through re-admissions, for example.

Manufacturers of medical therapy supplies and equipment are also under pressure from the care providers to provide therapy supplies and equipment with a focus on outcomes and costs. As therapy supplies and equipment are deployed to care providers, the manufacturers are challenged to provide evidence-based support for the care providers to justify the deployment of the therapy supplies and equipment by the care provider. This evidence-based approach is exacerbated by the variations of the medical profile variation of the population served by specific care providers. While certain therapies and equipment might provide a particular cost-benefit ratio in one population, another population may not experience the same cost-benefit ratio.

Variations in the medical profile of patient populations are often based on environmental and socioeconomic factors in the population served by a particular care provider. For example, in a particular area, the profile of the population of the area may result in a higher incidence of obesity, while in another area, obesity is not a significant factor in morbidity. In still another area, the environment may result in an increased incidence of respiratory illness.

Care providers are challenged to provide effective care across their entire population, but may experience different drivers of cost based on the population, and therefore may require different types of therapy supplies and equipment to address the costs of the specific population. Still further, if the care provider is successful in improving outcomes, the risk profile of the particular population may change over time such that most effective therapies and equipment changes based on changes in the patient population.

Still further, medical products may be configured to provide prophylactic and/or therapeutic care crosses over multiple patient diagnoses with varying efficacy. For example, a type of medical product may have multiple features, with each feature addressing a particular diagnoses or providing prophylactic support for hospital acquired injuries or conditions. The differences between efficacies across multiple products introduces variations in the outcomes expected for various potential diagnoses. Still further, multiple types of medical products may be applied to a particular patient with the medical products having a cumulative effect on therapeutic care and/or prophylaxis.

With these ongoing challenges, there is a need for a system and method that provides a cost effective approach to identification, deployment, and application of medical products across a particular populations to optimize the cost benefit and which is responsive to changes in the patient population over time.

### SUMMARY

The invention is defined in the independent claims. Embodiments of the invention are set out in the dependent claims.

According to a first aspect of the present disclosure, a method of reducing the cost of delivery of medical care in a population is disclosed. The method includes establishing a unique clinical profile for each of a plurality of different type of medical products of a fleet of disparate medical products, the clinical profile correlating the type of medical product to each particular clinical diagnosis affected by the type of medical product. The method also includes establishing, for each particular type medical product, an effectiveness factor for each clinical diagnosis affected by the type of medical product. The method further includes establishing, for each particular medical product, a cost factor for each clinical diagnosis. The method still further includes establishing a population profile for a particular population, the population profile identifying an incidence rate for each of the clinical diagnoses affected by the fleet of disparate medical products. The method still yet further includes optimizing, by a computer processor, a group of medical products to be deployed for treatment of the population by maximizing the effectiveness of the group of medical products while minimizing the total cost of the selected group of medical products. The method also includes generating, by a computer processor, a deployment schedule for deployment of the optimized group of medical products for treatment of the particular population.

The method of the first aspect of the present disclosure may further comprise deploying the optimized group of medical products to a treatment facility to provide the appropriate inventory of medical products to be used to treat the particular population.

The method of the first aspect of the present disclosure may further comprise choosing, based on the profile of clinical diagnoses of a particular member of the population, a subset of medical products that are selected from the optimized group of medical products, the subset being chosen to provide the highest effectiveness for the profile of clinical diagnoses of the particular member of the population, to thereby effectively treat the particular member of the population.

The method of the first aspect of the present disclosure may further comprise modifying, based on an updated profile of clinical diagnoses of the particular member of the population after a period of time, the subset of medical products to continue to effectively treat the particular member of the population at a lowered cost.

In some embodiments of the first aspect of the present disclosure, the cost of the type of medical product includes an analysis of a cost offset of a payment by a third party which lowers the cost to the provider of the treatment.

In some embodiments of the first aspect of the present disclosure, the effectiveness of the type of medical product includes a factor that considers the prophylactic impact of the type of medical product on a particular clinical diagnosis.

In some embodiments of the first aspect of the present disclosure, the effectiveness of the type of medical product includes a factor that considers the therapeutic impact of the type of medical product on a particular clinical diagnosis.

In some embodiments of the first aspect of the present disclosure, the effectiveness of the type of medical product includes a factor that considers the expected length of stay at a medical treatment facility by the particular member of the population.

The method of the first aspect of the present disclosure may further comprise treating the particular population using the optimized group of medical products.

According to a second aspect of the present disclosure, a computer program product for reducing the cost of delivery of medical care in a population comprises a non-transitory computer readable storage medium having program instructions embodied therewith, the program instructions executable by a computer processor to cause the computer processor to perform a method. The method includes establishing a unique clinical profile for each of a plurality of different type of medical products of a fleet of disparate medical products, the clinical profile correlating the type of medical product to each particular clinical diagnosis affected by the type of medical product. The method also includes establishing, for each particular medical product, an effectiveness factor for each clinical diagnosis affected by the type of medical product. The method further includes establishing, for each particular medical product, a cost factor for each clinical diagnosis. The still further includes establishing a population profile for a particular population, the population profile identifying an incidence rate for each of the clinical diagnoses affected by the fleet of disparate medical products. The method yet further includes optimizing a group of medical products to be deployed for treatment of the population by maximizing the effectiveness of the group of medical products while minimizing the total cost of the selected group of medical products. The method yet still further includes generating a deployment schedule for deployment of the optimized group of medical products for treatment of the particular population.

In some embodiments of the second aspect, the computer program product further comprises program instructions for deploying the optimized group of medical products to a treatment facility to provide the appropriate inventory of medical products to be used to treat the particular population.

In some embodiments of the second aspect, the computer program product further comprises program instructions for choosing, based on the profile of clinical diagnoses of a particular member of the population, a subset of medical products that are selected from the optimized group of medical products, the subset being chosen to provide the highest effectiveness for the profile of clinical diagnoses of the particular member of the population, to thereby effectively treat the particular member of the population.

In some embodiments of the second aspect, the computer program product further comprises program instructions for modifying, based on an updated profile of clinical diagnoses of the particular member of the population after a period of time, the subset of medical products to continue to effectively treat the particular member of the population at a lowered cost.

In some embodiments of the second aspect, the cost of the type of medical product includes an analysis of a cost offset of a payment by a third party which lowers the cost to the provider of the treatment.

In some embodiments of the second aspect, the effectiveness of the type of medical product includes a factor that considers the prophylactic impact of the type of medical product on a particular clinical diagnosis.

In some embodiments of the second aspect, the effectiveness of the type of medical product includes a factor that considers the therapeutic impact of the type of medical product on a particular clinical diagnosis.

In some embodiments of the second aspect, the effectiveness of the type of medical product includes a factor that considers the expected length of stay at a medical treatment facility by the particular member of the population.

A computer system for reducing the cost of delivery of medical care in a population comprises one or more computer processors, one or more computer readable storage media, program instructions stored on the computer readable storage media for execution by at least one of the one or more processors. The program instructions comprise instructions for establishing a unique clinical profile for each of a plurality of different type of medical products of a fleet of disparate medical products, the clinical profile correlating the type of medical product to each particular clinical diagnosis affected by the type of medical product. The computer instructions further comprise instructions for establishing, for each particular medical product, an effectiveness factor for each clinical diagnosis affected by the type of medical product. The computer instructions still further comprise instructions for establishing, for each particular medical product, a cost factor for each clinical diagnosis. The computer instructions still yet further comprise instructions for establishing a population profile for a particular population, the population profile identifying an incidence rate for each of the clinical diagnoses affected by the fleet of disparate medical products. The computer instructions also comprise instructions for optimizing a group of medical products to be deployed for treatment of the population by maximizing the effectiveness of the group of medical products while minimizing the total cost of the selected group of medical products. The computer instructions also yet further comprise instructions for generating a deployment schedule for deployment of the optimized group of medical products for treatment of the particular population.

In some embodiments of the third aspect, the program instructions comprises instructions for choosing, based on the profile of clinical diagnoses of a particular member of the population, a subset of medical products that are selected from the optimized group of medical products, the subset being chosen to provide the highest effectiveness for the profile of clinical diagnoses of the particular member of the population, to thereby effectively treat the particular member of the population.

In some embodiments of the third aspect, the program instructions comprises instructions for modifying, based on an updated profile of clinical diagnoses of the particular member of the population after a period of time, the subset of medical products to continue to effectively treat the particular member of the population at a lowered cost.

In some embodiments of the third aspect, when evaluating the cost of a type of medical product the program instructions include an analysis of a cost offset of a payment by a third party which lowers the cost to the provider of the treatment.

In some embodiments of the third aspect, when evaluating the effectiveness of the type of medical product, the computer instructions include a factor that considers the prophylactic impact of the type of medical product on a particular clinical diagnosis.

In some embodiments of the third aspect, when evaluating the effectiveness of the type of medical product, the computer instructions include a factor that considers the therapeutic impact of the type of medical product on a particular clinical diagnosis.

In some embodiments of the third aspect, when evaluating the effectiveness of the type of medical product, the computer instructions include a factor that considers the expected length of stay at a medical treatment facility by the particular member of the population.

Additional features, which alone or in combination with any other feature(s), such as those listed above and/or those listed in the claims, can comprise patentable subject matter and will become apparent to those skilled in the art upon consideration of the following detailed description of various embodiments exemplifying the best mode of carrying out the embodiments as presently perceived.

### BRIEF DESCRIPTION OF THE DRAWINGS

The detailed description particularly refers to the accompanying figures in which:
Fig. 1 is a diagrammatic representation of a health care system according to the present disclosure;
Fig. 2 is a diagrammatic representation of a portion of a healthcare delivery model that utilizes data the effectiveness and costs of using medical products to optimize a group of medical products to treat a specific patient population;
Figs. 3A-3C are diagrammatic representations of medical products having differing features that may be used to treat a specific patient;
Fig. 4 is a diagrammatic representation of the elements used to operate the healthcare delivery model of Fig. 2;
Fig. 5 is a table illustrating a database of patient population diagnostic information used in an embodiment of the healthcare delivery model of Fig. 2;
Fig. 6 is a table illustrating a database of the daily cost of a group of products and the efficacy of the products relative to certain diagnostic codes, the database used in an embodiment of the healthcare delivery model of Fig. 2; and
Fig. 7 is a table illustrating a database of the risk factors associated with respective diagnostic codes, the risk factors of the database used in an embodiment of the healthcare delivery model of Fig. 2 to adjust a daily cost of a product that impacts the diagnostic code.

### DETAILED DESCRIPTION

According to the present disclosure, diagnostic code(s) associated with the therapeutic or prophylactic capabilities of medical products are established and cohorts of patients that have the associated diagnostic code(s) in their patient records are compared to establish an optimized medical product deployment and implementation strategy that leverages medical product efficacy to provide effective prophylactic and therapeutic care. Patient level data is segregated into patients with hospital acquired conditions and environmentally acquired conditions, represented by the diagnostic codes. Creation of these cohorts and the comparative analysis of outcomes to medical products establishes the impact of medical products clinically and financially to an organization to manage the cost of efficacious care.

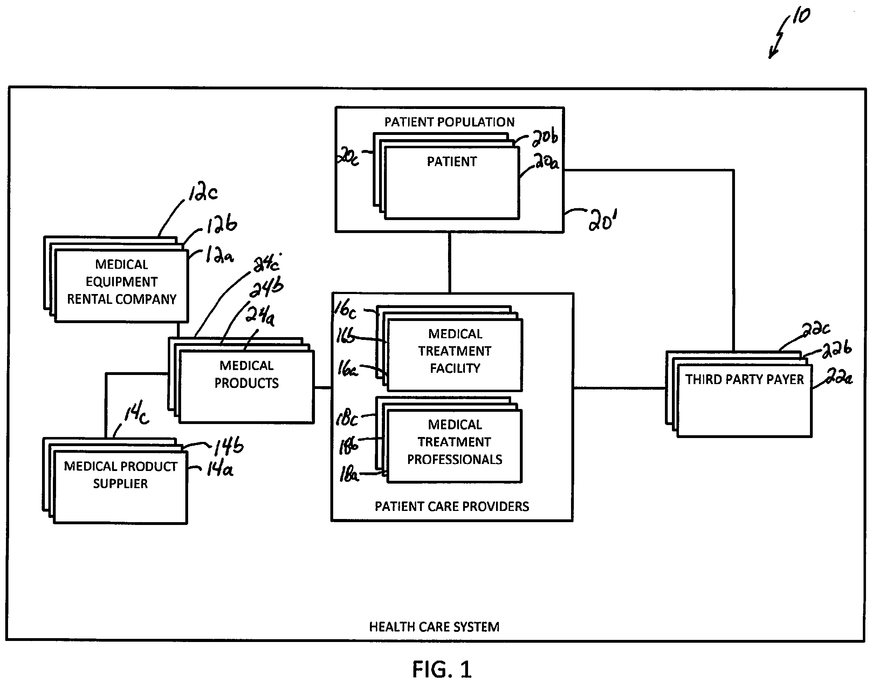

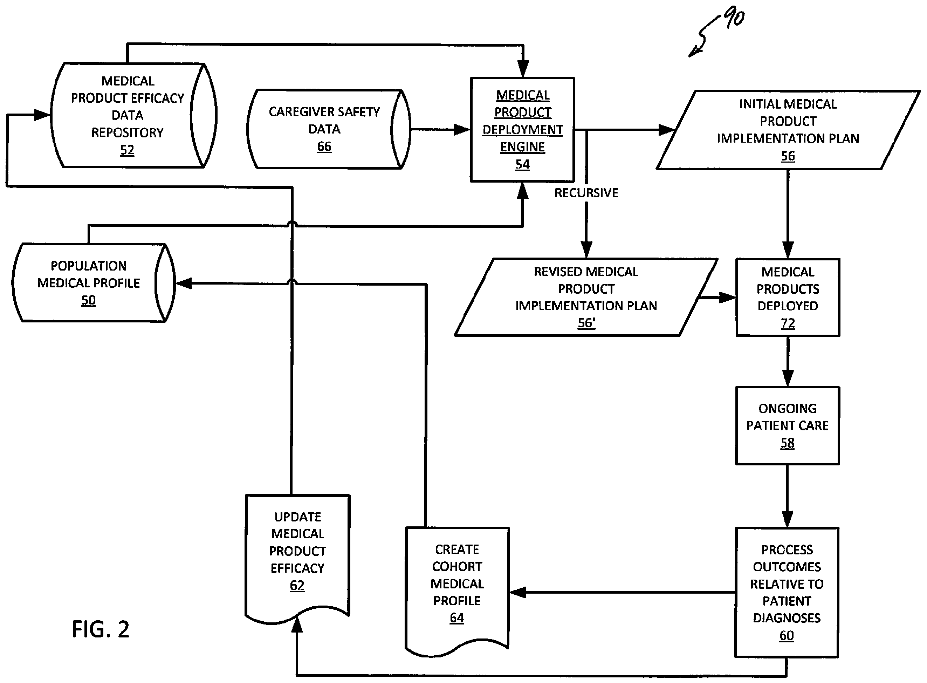

For example, Fig. 1 illustrates actors that participate in a healthcare delivery model 90 (see Fig. 2) for delivery of healthcare in a healthcare system 10 that treats patients 20a, 20b, 20c. The illustrative system 10 includes a plurality of medical product suppliers 12a, 12b, and 12c, a plurality of medical equipment rental companies 14a, 14b, 14c, a system of medical treatment facilities 16a, 16b, 16c, a plurality of medical treatment professionals 18a, 18b, 18c, a patient population 20', that includes a plurality of patients 20a, 20b, 20c, and a plurality of third party payers 22a, 22b, 22c. Each of the medical treatment facilities 16a, 16b, 16c or medical treatment professionals 18a, 18b, 18c may be referred to individually as a patient care provider. In the following discussion, when referred in the singular, reference will be made to a medical product supplier 12, a medical equipment rental company 14, a medical treatment professional 18, a patient 20, and a third-party payer 22.

A medical product supplier 12 may provide any type of medical product used by a facility to provide care to a patient 20. In some cases, a medical product 24 includes a capital product acquired by the medical treatment facility 16 and used for providing care. Simple examples of such products includes: diagnostic equipment such as x-ray cameras or CT scanners; re-usable patient support apparatuses such as stretchers or beds; treatment equipment such as infusion pumps or respiratory therapy devices. In other examples, a medical treatment product 16 includes products consumed in the treatment or prophylaxis of a patient. For example, a medical treatment product 16 includes medications, diagnostic leads, medical implants, pacemakers, or other patient related consumables. For purposes of this disclosure, medical treatment product 16 includes any product used in the treatment or prophylaxis of a patient and for which the medical treatment facility 16 incurs a cost for providing care to the patient 20.

Third-party payers 22a, 22b, 22c include any entity that is involved in paying the treatment facility 16 for treatment received by a respective patient 20. Third-party payers 22a, 22b, 22c include Medicare, the Veterans Administration, commercial insurers, employers, or any other party that reimburses and manages healthcare expenses. Third-party payers 22a, 22b, 22c often take an active role in determining the eligibility for reimbursement of certain expenses incurred by a healthcare facility 16. Third-party payers 22a, 22b, 22c often enter into contracts with medical treatment facilities 16 that limit the amount of reimbursement for expenses incurred by the medical treatment facility 16 to limit the third-party payers 22a, 22b, 22c exposure to expenses and charges. There are a multitude of pay arrangements that may be entered into between medical treatment facilities 16 and third-party payers 22a, 22b, 22c, all with the aim of encouraging medical treatment facilities 16 to manage and control the expenses of healthcare provided to the patients covered by the respective third-party payer 22. This makes it very important for the medical treatment facility 16 to determine the health profile of the patient population 20' served by the medical treatment facility 16 to understand the various disease modalities experienced by the patient population 20' so that the medical treatment facility 16 can negotiate an appropriate reimbursement profile. Over time, third-party payers 22a, 22b, 22c have also looked to patient populations 20' to determine their own risk in taking on coverage of a particular patient population 20'. As the third-party payers 22a, 22b, 22c and medical treatment facilities 16 have begun to view patient populations 20' in totality, the need to drive for improved care delivery to reduce overall cost of healthcare delivery has caused medical treatment facilities 16 to look to medical product suppliers 12 and medical equipment rental companies 14 to justify the cost of their products and to undertake risk sharing with the medical treatment facilities 16.

The present disclosure is directed to an approach to be used by medical product suppliers 12a, 12b, 12c and medical equipment rental companies 14a, 14b, 14c to provide cost justification to medical treatment facilities 16a, 16b, 16c and engage in risk management and risk sharing with the medical treatment facilities 16a, 16b, 16c to help improve the efficacy and control the costs of the delivery of healthcare over time. In short, variations in the diagnoses and morbidity of a patient population 20' varies from that of a larger population. An ability to refine the modality of care to the particular patient population 20', and even more specifically to particular patients 20a, 20b, 20c as disclosed herein provides an opportunity to improve outcomes and control costs while continuously refining the medical products 24 used in patient care. Refining treatment modalities allows a medical treatment facility 16 to optimize the purchase and deployment of medical products 24a, 24b, 24c to account for the specific needs of the patient population 20' and, more specifically, the particular patients 20a, 20b, 20c. This optimization provides for minimization of the costs of the delivery of healthcare in the particular patient population 20'. While the primary driver in any healthcare delivery model is to provide the best care available, there is a need to do so at the best cost return. This allows a particular medical treatment facility 16 to contract with a third-party payer 22 with improved accuracy so that the medical treatment facility 16 is financially incented to control costs, ultimately reducing the costs incurred by the third-party payers 22a, 22b, 22c.

The challenge in such a situation is to have accurate data to use in determining the appropriate medical products 24a, 24b, 24c for a particular patient population 20' in a particular medical treatment facility 16. Aggregated data from health studies or from a third-party payer 22 must be applied by the medical treatment facility 16 with an assumption of a wide measure of variability to account for the staleness of the data, ongoing changes in the demographics of the patient population 20' being treated by the medical treatment facility 16, or changes in lifestyle choices within the patient population 20'. More importantly, what is missing from traditional approaches, is the lack of closed-loop feedback to account for the effect of various medical products 24a, 24b, 24c on outcomes. While patient care occurs in real time, outcomes and efficacy have to be studied historically to account for changes in the impact of various care plans and treatment modalities.

The present disclosure utilizes an ongoing closed-loop analysis of the efficacy of medical products 24a, 24b, 24c on individuals in the patient population 20', along with real-time assessments of the changes in the health profile of the patient population 20' to continuously monitor and refine the treatment modalities and the medical products 24a, 24b, 24c deployed for treatment and prophylaxis of the patient population 20' to provide the group of medical products 24a, 24b, 24c to be deployed for treatment of the population 20' by maximizing the effectiveness of the group of medical products 24a, 24b, 24c while minimizing the total cost of the selected group of medical products 24a, 24b, 24c to reduce the cost of treatment and prophylaxis.

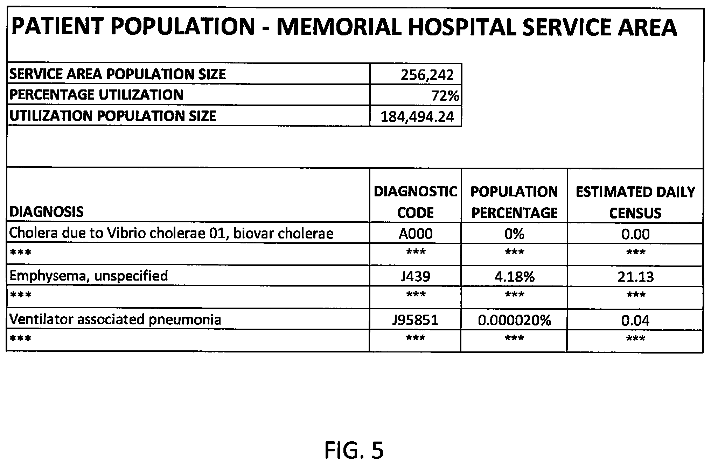

To accomplish this, the patient population 20' to be considered in the analysis must be established. Referring to Fig. 2, a population medical profile 50 is established and stored by considering an appropriate time series of the International Classification of Diseases ("ICD") codes of patients treated by the medical treatment facility 16 correlated with other pertinent demographic data to establish a population medical profile 50 of injuries and diseases of the patient population 20' being treated by the medical treatment facility 16. To the extent that the medical treatment facility 16 has multiple facilities and/or locations, the population medical profile 50 correlates that information as well. Referring now to Figs. 2 and 5, the medical profile 50 of the population 20' is established using the appropriate data sources for the population to be considered, such as information from the third-party payers, information from the healthcare facility medical records, public or private data sets such as datasets from Definitive Healthcare, LLC, the Healthcare Cost and Capitalization Project data from the Agency for Healthcare Research and Quality under the U.S. Department of Health & Human Services, research papers, or any other reasonable source of data that will provide pertinent information regarding the disease, injury, and morbidity profile of the population 20'. This information is logically associated along with other demographic data in the population medical profile 50.

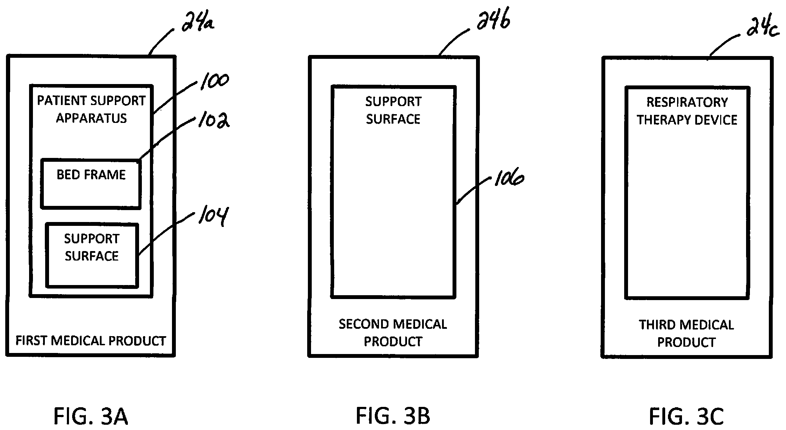

In addition to the population medical profile 50, an initial medical product efficacy profile 52 of medical products 24a, 24b, 24c available to the medical treatment facility 16 is established and stored. For example, each medical product 24a, 24b, 24c is associated with particular ICD codes and/or Current Procedural Terminology (CPT^{®}) codes that are associated with the medical products 24. A medical product efficacy data repository 52 is also created by associating the ICD codes, CPT codes, diagnostic related group (DRG) codes (Codes) and the related efficacy and cost data associated with each medical product to be analyzed. For example, Fig. 3 shows a table that correlates particular products, Product 1, Product 2, Product 3 and extending to Product n, which represents that any number of products may be included in the correlation, to Code 1, Code 2, Code 4 and extending to Code n. The correlation shows the efficacy of each Product 1-n in addressing diagnoses or treatments associated with Codes 1-n. It should be understood that medical products 24a, 24b, 24c may be associated with any given code and a particular medical product 24 may be associated with multiple Codes. For example, when treating a particular disease state, a first medical product 24a capable of treating the particular disease state, also treats additional disease states. This first medical product 24a has expanded applicability. Examples of multiple medical products 24a, 24b, 24c having efficacy for particular ICD or CPT codes is shown in Figs. 3A-3C. In the example of Fig. 3A, the first medical product 24a comprises a patient support apparatus 100 that includes a dedicated bed frame 102 and a dedicated support surface. 104. The support surface 104 is configured to provide both pulmonary therapy for patients at risk of developing pneumonia and low pressure therapy for patients at risk of developing pressure ulcers. For example, the patient support apparatus 100 may be embodied as a Progressa^{®} bed with therapy surface, available from Hill-Rom of Batesville, IN. As shown in Fig. 3B, a second medical product 24b is a support surface 106 that is configured as a mattress replacement system that is configured to provide only low pressure therapy. For example, the medical product 24b is a medical product type that may be embodied as a Hill-Rom^{®} 300 Wound Surface, also available from Hill-Rom. The second medical product 24b is configured to be placed on any of a number of different configurations of bed frames. Thus, the second medical product 24b includes one modality that overlaps with the modalities of the first medical product 24a. This provides a simple example of how two different medical products 24a, 24b, 24c have overlapping modalities of treatment. In addition, they have different associated costs of acquisition and operation. Still further, they each have a different efficacy for each of their modalities of treatment. This creates a composite score for cost of delivering the modality.

For example, medical product 24a has an efficacy factor for respiratory therapy, an efficacy factor for low pressure therapy, and a daily cost of operation. These values may be applied in any of a number of ways to establish composite score that identifies an effective daily cost of operation of the type of medical product 24a to provide the two modalities. However, if a patient does not require a particular modality, the effective cost of operation is adjusted by the efficacy of the one modality and the capability of providing the second modality is lost due to the lack of need of the second modality. Thus, underutilizing the modalities that a medical product 24 is capable of increases the net cost of operation of that medical product 24. Thus, while both medical product 24a and medical product 24b are capable providing low pressure therapy, if the daily cost of operation of medical product 24a is much higher than the daily cost of operation of medical product 24b and a patient only need low pressure therapy, it is likely that medical product 24a will be the more expensive medical product 24 to use.

Yet another medical product 24c is configured to only provide respiratory therapy. For example, medical product 24c may be embodied as a Monarch^{®} System available from Hill-Rom, which is capable of providing respiratory therapy. In some cases, the medical product 24b and medical product 24c may be used simultaneously to provide both low pressure therapy and respiratory therapy. However, the efficacy of each of medical product 24b and medical product 24c are different for their respective modalities and different from the medical product 24a with respect to those modalities.

While this is an elementary explanation of the how different medical products 24a, 24b, 24c each have different costs of use and efficacy, the person of ordinary skill will understand that many medical products have overlapping an multiple modalities or uses. Different patient populations 20' have different health profiles such that the appropriate mix of deployment of the medical products 24a, 24b, 24c varies from population 20' to other populations to achieve the lowest cost of ownership/deployment, while still providing the appropriate medical products 24a, 24b, 24c to adequately care for the individual patients 20a, 20b, 20c, in the population.

The information from the population medical profile 50 and the medical product efficacy data repository 52 is fed to a medical product deployment engine 54 that is operable to consider the cost of operation of the respective medical products along with the efficacy of the particular modalities to create a cost function for each modality of each medical product 24 and population medical profile 52 establish an initial medical product implementation plan 56. By applying the initial medical product implementation plan 56, the healthcare facility 24 creates a standard inventory of medical products 24. The initial medical product implementation plan 56 further includes decision criteria for which medical products 24 to apply to a particular patient profile based on the Codes associated with the patient being treated. It should be understood that the initial product implementation plan 56 to have contingent deployment schemes that may be used if a primary employment scheme for a particular patient is not available due to medical products 24 already being deployed with other patients. Additionally, if an appropriate medical product 24 is not readily available for a particular patient, the initial medical product implementation plan 56 may include a plan to acquire an appropriate medical product 24 from an external source such as a medical equipment rental company 14a, 14b, 14c or from a medical product supplier 12a, 12b, 12c. For example, data may be automatically transmitted to the external source indicating the required medical product to cause that required medical product to be supplied.

Once the appropriate medical products 24 have been identified and deployed to particular patients 20a, 20b, 20c as indicated at 72 in Fig. , the deployed medical products 24a, 24b, 24c are used provide ongoing patient care at 58. Importantly, the outcomes relative to the provided patient care are measured at 60 and correlated to ICD codes, DRGs, or CPT codes. Because the actual outcomes of the healthcare facility become available after outcomes relative to the patient diagnoses are processed at 60, medical product efficacy is updated at repository 62 and is applied to medical product efficacy data repository 52. Additionally, the population medical profile 50 is supplanted with information from the processing step 60 cohort medical profile 64. The updated data from the profile 64 and the repository 62 is used to update the operation of the medical product deployment engine 54 which recursively revises the medical product implementation plan based on results data so that actual, real-time, patient outcomes can be used to further optimize the delivery of patient care to form a revised medical product implementation plan 56'.

In addition to patient outcome data, caregiver safety data may be stored in an optional repository 66, the caregiver safety data being used by the medical product employment engine 54 to modify the cost associated with a particular medical product 24 based on a risk of injury to a caregiver. In such a case, the delivery of healthcare must be considered to include the risks of injury, such as when a caregiver has to manually reposition the patient, as compared to a deployment plan that includes medical products 24, such as a patient lift, for example, that will reduce the risk of injury to a caregiver, and thereby lower the cost of the delivery of care.

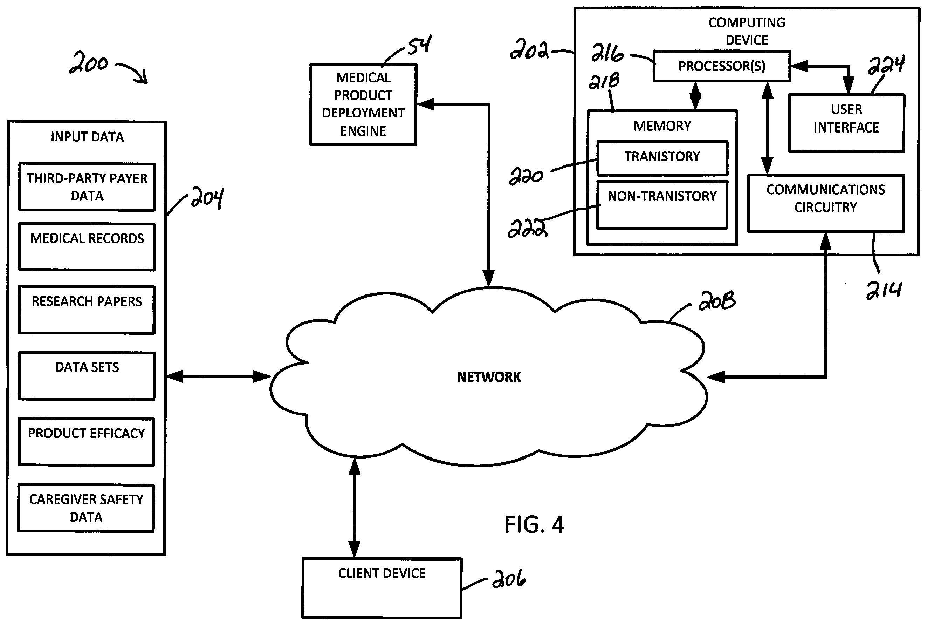

In one embodiment, the present disclosure may be implemented in a distributed data processing environment 200 as illustrated in Fig. 4. The distributed data processing environment 200 includes computing device 202, the medical product deployment engine 54, input data 204, a client device 206, all connected through a network 208.

Network 208 can be, for example, a local area network (LAN), a wide area network (WAN), such as the Internet, or a combination of the two, and can include wired or wireless connections.

Network 208 can be any combination of connections and protocols that will support communications via various links between computing device 202, input data 204, and the engine 54, as well as various components and devices associated with computing device 202 within distributed processing environment 200. The computing device 202 includes communications circuitry 214, a processor 216, memory 218 including transitory memory 220 and non-transitory memory 222, and a user interface 224. Instructions for performing the various computer based tasks and processes disclosed herein are stored in non-transitory memory 222.

The medical product deployment engine 54 is operable to make use of the components and devices associated with computing device 202 to perform analytical analysis under the direction of a user and to provide scenario analyses based on the input data as directed by a user. For example, a remote user, such as an analyst at a medical product supplier 12 or at a medical care facility 16 may change the assumptions used by the medical product deployment engine 54 in developing the medical product implementation plan 56. For example, the statistical variability applied to a population 20' may be changed to provide a what-if analysis for the user to understand the sensitivity of the medical product implementation plan 56 to potential variations in the diagnoses of the population 20'.

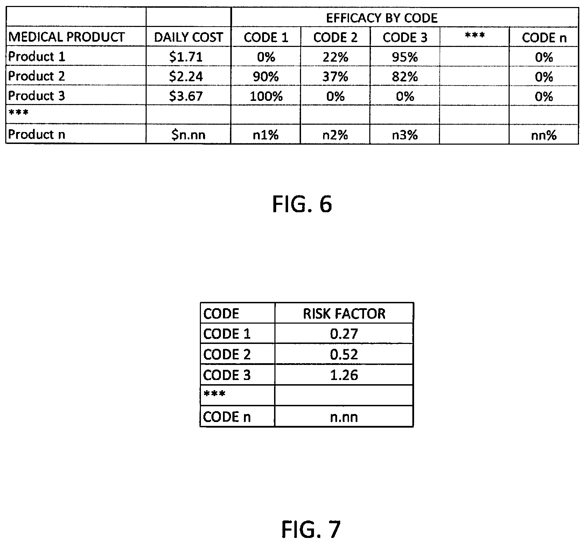

Referring now to the tables in Figs. 5-7, a set of conditions for a hypothetical example that includes a set of Codes associated with a group of and medical products is presented. Fig. 5 presents an example for how the data for a patient population 20' may be organized. Fig. 6 presents a table that may be used to document a group of 1 to n medical products and includes the daily cost of those products and the efficacy for each of the products over a number of Codes. Fig. 7 provides a relative risk of each of the Codes. In practice, the risk scores provide a normalized analysis of the risk of each of the Codes to expenses in the facility. The risk factor is a normalized measure of the expense to the medical facility of treating the patient having the Code. In some instances, this could simply be in dollars. It will be seen in the present examples that the normalized risk may be considered to compare the cost of different medical products in treating the illness or injury associated with the Code.

Referring to Fig. 5, input data 204 includes the population medical profile 50 which includes a summary of the patient population 20' that includes the incidence and estimated daily census for particular diagnosis as shown in Fig. 5. Using this data as an initial seed into the medical product deployment engine 54 considers the profile of the specific patient population 20' in determining the initial medical product implementation plan 56. In determining the medical product implementation plan 56, the medical product deployment engine 54 considers the data from population medical profile 50 and medical product efficacy data repository 52, the determines the equipment needs of the particular medical provider 62 by establishing a target census based on statistical parameters. Once the target census is established, the medical product deployment engine 54 performs an analysis to minimize the costs of medical products to be deployed to the care provider 62 to develop the medical product implementation plan 56. Referring to the generalized discussion of Figs. 3A-3B, a more specific hypothetical description is provided below.

Referring to Figs. 6-7, for a first example, consider a case where a first patient has been diagnosed with the condition associated with Code 3. It is easy to see that Product 1 has both a higher efficacy (95%) and a lower cost ($1.71 vs. $2.24) than Product 2 for addressing a diagnosis Code 3. Thus, it would be a simple choice to apply Product 1 to the care for the first patient with no detailed analysis as regardless of the risk of Code 3, Product 1 is the better choice.

In a second example, consider a situation where a second patient has been diagnosed with conditions associated with both Codes 2 and 3. The decision here is not a clear as the variations in the Risk Factor, efficacy, and Daily Cost require further analysis. In considering Product 1, a risk and efficacy based adjustment to the Daily Cost can be analyzed by modifying the Risk Factor by the efficacy to adjust the Risk Factor based on the efficacy of the product. By calculating an efficacy based inflator for the Risk Factor, the Risk Factor can be adjusted for the product. The lower the efficacy, the more the Risk Factor should be inflated. As such, a simple step of dividing the Risk Factor by the efficacy should have the desired impact on inflating the Risk Factor. If a product is 100% efficacious, then the Risk Factor will not be inflated in any way.

For Product 1, Code 2, the Risk Factor of 0.52 should be adjusted by dividing the Risk Factor by the efficacy of 22% to get an adjusted Risk Factor of 2.36. Thus, the Daily Cost of $1.71 Product 1 would be inflated by 2.36 to an adjusted value of $4.04 for Code 2. For Product 2, Code 3, the Risk Factor of 1.26 should be adjusted by dividing the Risk Factor by the efficacy of 95% to get an inflated Risk Factor of 1.33. Thus, adjusted Daily Cost of $1.71 for Product 1 for Code 3 is $2.27. Adding the Code 2 and Code 3 relative costs results in a total adjusted cost of Product 1 for treating Code 2 and Code 3 is $6.31.

Performing the same analysis for Product 2 results in inflating the Risk Factor for Code 2 by dividing 0.52 by 37% to get 1.41, which when multiplied by the Daily Cost of $2.24 results in an adjusted Daily Cost of $3.15. This compares favorably to the adjusted Daily Cost of Code 2 for Product 1 of $4.04 because the higher efficacy offsets the higher cost. With regard to Code 3 for Product 2, the risk factor of 1.26 is inflated by dividing it by 82% to get an inflated risk factor of 1.54. Multiplying the inflated risk factor by the Daily Cost for Product 3, the Code 3 inflated Daily cost is $3.45 which compares unfavorably to the Code 3 inflated daily cost of Product 1 of $2.27. The combined cost of Product 2 for Codes 2 and 3 is $6.60 which is more that the cost of Product 1 of $6.31. Thus, Product 1 would be chosen for treating a patient diagnosed with both Codes 2 and 3.

In a third example, consider a third patient that is diagnosed with Codes 1, 2, and 3. In this case, Product 1 does not treat all three Codes. However, Product 2 does treat all three Codes. However, consider that Product 3 may be used in conjunction with Product 1 such that the two products combine to treat Codes 1, 2, and 3. This combination can be compared to Product 2 to determine whether the combination of Products 1 and 3 results in a lower adjusted Daily Cost than Product 2.

As noted above, the total cost of Product 1 for Codes 2 and 3 is $6.31. Thus, looking to the impact of Code 1, it is seen that Product 3 is 100% efficacious. Thus, the Daily Cost of $3.67 of Product 3 is simply multiplied by the Risk Factor of 0.27 to arrive at an adjusted Daily Cost of $0.99. When added to the $6.31 for Product 1 for Codes 2 and 3, it can be seen that the total adjusted Daily Cost of treating Codes 1, 2, and 3 with Products 1 and 3 is $7.30.

The cost of treating Code 1 with Product 2 is calculated by inflating the Code 1 Risk Factor of 0.27 by the efficacy of 90% to get an inflated Risk Factor of 0.3. Multiplying the 0.3 by the $2.24 Daily Cost of Product 2 for Code 1 results in an adjusted Daily Cost of $0.67 for Product 2 to treat Code 1. Adding the $0.67 to the $6.60 calculated above for Product 2 for the adjusted Daily Cost of Codes 2 and 3, the total adjusted Daily Cost for Product 2 for Codes 1, 2, and 3 results in $7.27, which compares slightly favorably to the combination of Products 1 and 3 which had an adjusted Daily Cost of $7.30.

The foregoing three hypothetical examples show how the relative costs of particular products may be compared to minimize the cost of treating a particular combination of diagnoses. However, other factors may also be brought to bear in the analysis. For example, to be considered for a particular treatment, a product may be required to have a minimum efficacy such that a product may be disqualified for being considered for selection for a particular Code if the efficacy does not meet the minimum threshold.

As noted above with regard to the recursive approach of modifying the inputs to the medical product implementation plan 56, the equipment deployed for a particular patient may be updated based on an updated profile of clinical diagnoses of the particular patient after a period of time based on the medical product(s) 24 optimized to continue to effectively treat the particular member of the population at a lowered cost. It should be understood that the Daily Cost may be adjusted in some embodiments to account for any cost offset of a payment by a third party payer 22 which lowers the cost to the provider 62 of the patient. Another adjustment that may be included is a factor that adjusts Daily Cost by considering the prophylactic impact of the medical product 24 on a particular clinical diagnosis or potential injury. For example, a Daily Cost may be adjusted by considering the impact that the product has on the rate of acquisition of hospital acquired pneumonia or pressure ulcers. Thus, while a particular patient may not currently be diagnosed with such an illness or injury, the prophylactic benefit of the product may be considered in the analysis of the Daily Cost to finalize an adjusted Daily Cost. Similarly, the Daily Cost may be adjusted by considering the impact that the product has on the length of stay of a patient. For example, a product that provides functions that are unrelated to any particular Code, but that are found to correlate to a shorter length of stay, may justify a factor to adjust the Daily Cost to reflect the positive effect on a patient. For example, a product that provides improved mobility for a patient postoperatively may correlate to a shorter length of stay due to a patient's faster progression to full mobility. This may be factored into reducing the adjusted Daily Cost reflective of that factor.

While the idealized daily census shown in Fig. 3 provides a population baseline, the medical product deployment engine 54 applies an analysis of the overlapping diagnoses such as those described in the three examples above and applies statistical methods to assure that the medical product implementation plan 56 deploys sufficient medical products 24a, 24b, 24c to cover variations in the patient population that actually presents at the medical treatment facility 16 at a chosen statistical level, such as 90%, for example. The medical product deployment engine 54 can be adjusted to consider the ability of the medical treatment facility 16 to acquire needed medical products 24a, 24b, 24c in real-time from other providers, such as medical equipment rental companies 16a, 16b, 16c.

Employing a system of the present disclosure can be effective in relatively small healthcare facilities, but may be scalable to a healthcare system in a particular geographic location, a consortium of healthcare facilities over expanded geographic region, or even nationally, to provide a scheme for providing effective delivery of healthcare through proper deployment of medical products. In addition, the present disclosure may be used provide simulations or financial projections for medical products 24 such that a medical product supplier 12 may be able to justify incremental improvements in product efficacy by showing that improvements in efficacy will have a significant impact on outcomes such as a patient's length of stay, costs of care, and other more qualitative measures of effectiveness of particular medical products.

While the disclosure has been illustrated and described in detail in the drawings and the foregoing description, such illustration and description are to be considered illustrative or exemplary and not restrictive. The disclosure is not limited to the disclosed embodiments. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

Aspects of the disclosure are set out in the following numbered clauses:
Clause 1. A method of determining delivery of medical care in a population comprising:
   establishing and storing, in one or more databases, a unique clinical profile for each type of medical product of a plurality of different medical products of a fleet of disparate medical products, the clinical profile correlating the type of medical product to each particular clinical diagnosis affected by the type of medical product;
   establishing and storing, in the one or more databases, for each type of medical product, an effectiveness factor for each clinical diagnosis affected by the type of medical product;
   establishing and storing, in the one or more databases, for each type of medical product, a cost factor for each clinical diagnosis;
   establishing and storing, in the one or more databases, a population profile for a particular population, the population profile identifying an incidence rate for each of the clinical diagnoses affected by the fleet of disparate medical products;
   optimizing, by a computer processor, a group of medical products to be deployed for treatment of the population by maximizing the effectiveness of the group of medical products while minimizing the total cost of the selected group of medical products; and
   generating, by a computer processor, a deployment schedule for deployment of the optimized group of medical products for treatment of the particular population.
Clause 2. The method of clause 1, further comprising deploying the optimized group of medical products to a treatment facility to provide the appropriate inventory of medical products to be used to treat the particular population.
Clause 3. The method of clause 2, wherein deploying the optimized group of medical products to a treatment facility comprises transmitting, to each of one or more suppliers of the optimized group of medical products, at least a part of the deployment schedule corresponding to deployment of a part of the optimized group of medical products to be supplied by that supplier.
Clause 4. The method of any preceding clause, further comprising:
   receiving a profile of clinical diagnoses of a particular member of the population; and
   automatically identifying, based on the profile of clinical diagnoses of the particular member of the population, a subset of medical products that are selected from the optimized group of medical products, the subset being identified to provide the highest effectiveness for the profile of clinical diagnoses of the particular member of the population, to thereby effectively treat the particular member of the population; and storing the subset of medical products in the one or more databases.
Clause 5. The method of clause 4, further comprising:
   receiving an updated profile of clinical diagnoses of the particular member of the population after a period of time; and
   automatically modifying, in the one or more databases and based on the updated profile of clinical diagnoses of the particular member of the population, the subset of medical products to continue to effectively treat the particular member of the population at a lowered cost.
Clause 6. The method of any preceding clause, further comprising, prior to the step of establishing and storing, in one or more databases, a unique clinical profile for each type of medical product of a plurality of different medical products of a fleet of disparate medical products:
   receiving information relating to the plurality of different medical products of the fleet of disparate medical products;
   wherein the establishing and storing, in one or more databases, a unique clinical profile for each type of medical product of a plurality of different medical products of a fleet of disparate medical products is based on the received information.
Clause 7. The method of clause 6, further comprising:
   receiving updated information relating to at least one of the plurality of different medical products of the fleet of disparate medical products;
   automatically re-optimizing the group of medical products to be deployed for treatment of the population by maximizing the effectiveness of the group of medical products while minimizing the total cost of the selected group of medical products based on the updated information relating to at least one of the plurality of different medical products of the fleet of disparate medical products; and
   automatically generating a first updated deployment schedule for deployment of the re-optimized group of medical products for treatment of the particular population.
Clause 8. The method of any preceding clause, further comprising
   receiving one or more of: an updated effectiveness factor for each clinical diagnosis affected by the type of medical product, an updated cost factor for each clinical diagnosis, and an updated population profile for a particular population;
   automatically re-optimizing the group of medical products to be deployed for treatment of the population by maximizing the effectiveness of the group of medical products while minimizing the total cost of the selected group of medical products based on the one or more of: an updated effectiveness factor for each clinical diagnosis affected by the type of medical product, an updated cost factor for each clinical diagnosis, and an updated population profile for a particular population; and
   automatically generating a second updated deployment schedule for deployment of the re-optimized group of medical products for treatment of the particular population.
Clause 9. The method of any preceding clause, wherein the cost of the type of medical product includes an analysis of a cost offset of a payment by a third party which lowers the cost to the provider of the treatment.
Clause 10. The method of any preceding clause, wherein the effectiveness of the type of medical product includes a factor that considers the prophylactic impact of the type of medical product on a particular clinical diagnosis.
Clause 11. The method of any preceding clause, wherein the effectiveness of the type of medical product includes a factor that considers the therapeutic impact of the type of medical product on a particular clinical diagnosis.
Clause 12. The method of any preceding clause, wherein the effectiveness of the type of medical product includes a factor that considers the expected length of stay at a medical treatment facility by the particular member of the population.
Clause 13. The method of any preceding clause, further comprising treating the particular population using the optimized group of medical products.
Clause 14. A computer program product for determining delivery of medical care in a population, the computer program product comprising:
   a non-transitory computer readable storage medium having program instructions embodied therewith, the program instructions executable by a computer processor to cause the computer processor to perform the method according to any of clauses 1 to 13.
Clause 15. A computer system for determining delivery of medical care in a population, the computer system comprising:
   one or more computer processors;
   one or more computer readable storage media;
   program instructions stored on the computer readable storage media for execution by at least one of the one or more processors, the program instructions comprising instructions for causing the computer system to perform the method of any of clauses 1 to 13.

## Claims

1. A method of determining delivery of medical care in a population comprising:
establishing and storing, in one or more databases, a unique clinical profile for each type of medical product of a plurality of different medical products of a fleet of disparate medical products, the clinical profile correlating the type of medical product to each particular clinical diagnosis affected by the type of medical product;
establishing and storing, in the one or more databases, for each type of medical product, an effectiveness factor for each clinical diagnosis affected by the type of medical product;
establishing and storing, in the one or more databases, for each type of medical product, a cost factor for each clinical diagnosis;
establishing and storing, in the one or more databases, a population profile for a particular population, the population profile identifying an incidence rate for each of the clinical diagnoses affected by the fleet of disparate medical products;
optimizing, by a computer processor, a group of medical products to be deployed for treatment of the population by maximizing the effectiveness of the group of medical products while minimizing the total cost of the selected group of medical products; and
generating, by a computer processor, a deployment schedule for deployment of the optimized group of medical products for treatment of the particular population.

2. The method of claim 1, further comprising deploying the optimized group of medical products to a treatment facility to provide the appropriate inventory of medical products to be used to treat the particular population.

3. The method of claim 2, wherein deploying the optimized group of medical products to a treatment facility comprises transmitting, to each of one or more suppliers of the optimized group of medical products, at least a part of the deployment schedule corresponding to deployment of a part of the optimized group of medical products to be supplied by that supplier.

4. The method of any preceding claim, further comprising:
receiving a profile of clinical diagnoses of a particular member of the population; and
automatically identifying, based on the profile of clinical diagnoses of the particular member of the population, a subset of medical products that are selected from the optimized group of medical products, the subset being identified to provide the highest effectiveness for the profile of clinical diagnoses of the particular member of the population, to thereby effectively treat the particular member of the population; and
storing the subset of medical products in the one or more databases.

5. The method of claim 4, further comprising:
receiving an updated profile of clinical diagnoses of the particular member of the population after a period of time; and
automatically modifying, in the one or more databases and based on the updated profile of clinical diagnoses of the particular member of the population, the subset of medical products to continue to effectively treat the particular member of the population at a lowered cost.

6. The method of any preceding claim, further comprising, prior to the step of establishing and storing, in one or more databases, a unique clinical profile for each type of medical product of a plurality of different medical products of a fleet of disparate medical products:
receiving information relating to the plurality of different medical products of the fleet of disparate medical products;
wherein the establishing and storing, in one or more databases, a unique clinical profile for each type of medical product of a plurality of different medical products of a fleet of disparate medical products is based on the received information.

7. The method of claim 6, further comprising:
receiving updated information relating to at least one of the plurality of different medical products of the fleet of disparate medical products;
automatically re-optimizing the group of medical products to be deployed for treatment of the population by maximizing the effectiveness of the group of medical products while minimizing the total cost of the selected group of medical products based on the updated information relating to at least one of the plurality of different medical products of the fleet of disparate medical products; and
automatically generating a first updated deployment schedule for deployment of the re-optimized group of medical products for treatment of the particular population.

8. The method of any preceding claim, further comprising
receiving one or more of: an updated effectiveness factor for each clinical diagnosis affected by the type of medical product, an updated cost factor for each clinical diagnosis, and an updated population profile for a particular population;
automatically re-optimizing the group of medical products to be deployed for treatment of the population by maximizing the effectiveness of the group of medical products while minimizing the total cost of the selected group of medical products based on the one or more of: an updated effectiveness factor for each clinical diagnosis affected by the type of medical product, an updated cost factor for each clinical diagnosis, and an updated population profile for a particular population; and
automatically generating a second updated deployment schedule for deployment of the re-optimized group of medical products for treatment of the particular population.

9. The method of any preceding claim, wherein the cost of the type of medical product includes an analysis of a cost offset of a payment by a third party which lowers the cost to the provider of the treatment.

10. The method of any preceding claim, wherein the effectiveness of the type of medical product includes a factor that considers the prophylactic impact of the type of medical product on a particular clinical diagnosis.

11. The method of any preceding claim, wherein the effectiveness of the type of medical product includes a factor that considers the therapeutic impact of the type of medical product on a particular clinical diagnosis.

12. The method of any preceding claim, wherein the effectiveness of the type of medical product includes a factor that considers the expected length of stay at a medical treatment facility by the particular member of the population.

13. The method of any preceding claim, further comprising treating the particular population using the optimized group of medical products.

14. A computer program product for determining delivery of medical care in a population, the computer program product comprising:
a non-transitory computer readable storage medium having program instructions embodied therewith, the program instructions executable by a computer processor to cause the computer processor to perform the method according to any of claims 1 to 13.

15. A computer system for determining delivery of medical care in a population, the computer system comprising:
one or more computer processors;
one or more computer readable storage media;
program instructions stored on the computer readable storage media for execution by at least one of the one or more processors, the program instructions comprising instructions for causing the computer system to perform the method of any of claims 1 to 13.
